# FASCICULE DE BREVET EUROPEEN

(11) **EP 0 868 434 B1**
(45) Date de publication et mention de la délivrance du brevet: **09.10.2002**
(21) Numéro de dépôt: 96943157.6
(22) Date de dépôt: 20.12.1996
(51) Int. Cl.: C07K 16/42, A61K 39/395, A61K 47/48, A61K 51/10, G01N 33/53

(54) **ANTICORPS ANTI-IDIOTYPIQUES DU FACTEUR DE CROISSANCE ENDOTHELIALE VASCULAIRE ET LEUR UTILISATION COMME MEDICAMENTS**
VASKULARE ENDOTHELZELLE-WACHSTUMSFAKTOR ANTIIDOTYPISCHE ANTIKÖRPER UND IHRE VERWENDUNG ALS ARZNEIMITTEL
ANTI-IDIOTYPIC ANTIBODIES OF VASCULAR ENDOTHELIAL GROWTH FACTOR AND USE THEREOF AS DRUGS

(30) Priorité: 21.12.1995 FR 9515243
(43) Date de publication de la demande: 07.10.1998
(73) Titulaire: CENTRE NATIONAL DE LA RECHERCHE SCIENTIFIQUE (CNRS), 75016 Paris (FR)
(72) Inventeur: PLOUET, Jean, F-31400 Toulouse (FR); JONCA, Frédéric, F-31520 Ramonville-Saint-Agne (FR); ORTEGA, Nathalie, F-31400 Toulouse (FR); RUCHOUX, Marie-Magdeleine, F-37100 Tours (FR)
(74) Mandataire: Grosset-Fournier, Chantal Catherine
(86) Numéro de dépôt international: FR9602041
(87) Numéro de publication internationale: WO97023510

(56) Documents cités:
- EP-A- 0 502 416
- WO-A-94/11499
- JOURNAL OF CELLULAR BIOCHEMISTRY.SUPPLEMENT 18A, 1994, page 328 XP000602723 PLOUET ET AL: "VEGF DEPENDENT TUMORAL PROGRESSION: STIMULATION BY ANTI VEGF IDIOTYPIC ANTIBODIES"
- BIOCHEMICAL AND BIOPHYSICAL RESEARCH COMMUNICATIONS, vol. 187, 1992, pages 1579-1586, XP002013861 TERMAN ET AL: "IDENTIFICATION OF THE KDR TYROSINE KINASE AS A RECEPTOR FOR VASCULAR ENDOTHELIAL CELL GROWTH FACTOR" cité dans la demande
- CELL, vol. 72, 1993, pages 835-846, XP002013862 MILLAUER ET AL: " HIGH AFFINITY VEGF BINDING AND DEVELOPMENTAL EXRPESSSION SUGGEST FLK-1 AS A MAJOR REGULATOR OF VASCULOGENESIS AND ANGIOGENESIS" cité dans la demande
- NATURE, vol. 367, 1994, pages 576-579, XP002013863 MILLAUER ET AL: "GLIOBLASTOMA GROWTH INHIBITED IN VIVO BY A DOMINANT-NEGATIVE FLK-1 MUTANT" cité dans la demande
- NATURE, vol. 362, 1993, pages 841-844, XP002013864 JIN KIM ET AL: "INHIBITION OF VASCULAR ENDOTHELIAL GROWTH FACTOR-INDUCED ANGIOGENESIS SUPPRESSES TUMOUR GROWTH IN VIVO" cité dans la demande
- ANTI-CANCER DRUGS, vol. 5, 1994, pages 361-366, XP000601953 CHAGNAUD ET AL: "CURATIVE EFFECTS ON RAT SARCOMAS OBTAINED AFTER A TREATMENT COMBINING TWO MONOCLONAL ANTIBODIES"
- C.R.ACAD.SCI.PARIS,SCIENCE DE LA VIE, vol. 319, Mai 1996, pages 411-415, XP000601935 ORT GA ET AL: "MODULATION DE LA PROGRESSION TUMORALE PAR DES ANTICORPS ANTI-IDIOTYPIQUES DE FACTEURS ANGIOG NIQUES"

## Description

L'invention a pour objet des anticorps anti-idiotypiques du facteur de croissance endothéliale vasculaire et leur utilisation comme médicaments.

Le VEGF (vascular endothelial growth factor, c'est-à-dire : du facteur de croissance endothéliale vasculaire) est actuellement reconnu comme l'acteur majeur des néovascularisations non contrôlées observées dans la progression tumorale (revue dans Folkman), la rétinopathie diabétique (Malecaze, 1994) ou la polyarthrite rhumatoide (Fava, 1994), Il s'agit d'une glycoprotéine de 46 kDa se liant à l'héparine (Plouët Ferrara Connolly). Au moins 3 formes homodimères de 121, 165 et 189 acides aminés sont générées par épissage alternatif de l'ARN pré-messager. Le VEGF de 165 acides aminés sera désigné par VEGF 165. Outre son rôle angiogénique le VEGF stimule la perméabilité capillaire (Connolly, 1989). Le rôle du VEGF dans la tumorigénèse a été suspecté par l'observation que l'hypoxie accompagnant la nécrose tumorale augmentait l'expression de ses ARNm. D'autre part le VEGF est surexprimé dans de nombreux cas de pathologie tumorale. De plus l'injection d'anticorps anti-VEGF inhibe la croissance de tumeurs (Kim, 1993).

Or un facteur de croissance peut induire de nombreux effets différents selon qu'il se lie à l'un ou à l'autre de ses récepteurs, par exemple la prolifération et la survie. Donc l'immunoneutralisation peut avoir des effets bénéfiques en inhibant la prolifération mais aussi des effets indésirables en diminuant la survie. L'analyse des fonctions médiées *in vivo* par un récepteur de facteur de croissance se liant à l'héparine tels que le VEGF ou les FGFs (fibroblast growth factor) se heurte à deux écueils majeurs. D'une part la combinatoire des interactions entre ligands et récepteurs est fort complexe car un facteur de croissance peut se lier à plusieurs récepteurs et de même plusieurs facteurs de croissance peuvent se lier à un même récepteur. Ainsi les produits de 9 gènes codant pour les FGFs disposent des produits de 5 gènes codant pour des récepteurs des FGFs. D'autre part leur forte affinité pour les glycosaminoglycanes fait que les facteurs de croissance tels que VEGF ou FGF sont séquestrés dans les matrices extracellulaire et ne sont retrouvés qu'au voisinage immédiat (moins de 0,5 mm) de leur lieu de synthèse ce qui rend difficile l'appréhension de leur rôle *in vivo.*

Deux gènes différents codent pour des tyrosine kinases transmembranaires identifiées comme des récepteurs du VEGF : le KDR chez l'homme (Terman, 1992) ou le flk-1 chez la souris (Millauer, 1993) et le flt-1 (DeVries, 1992).

Il a été montré que les ARN messagers du récepteur flk-1 étaient présents dans les cellules endothéliales lors de l'embryogénèse et disparaissaient chez l'adulte (Millauer, 1993). Les seules cellules non endothéliales où le flk-1 a été retrouvé sont les cellules stromales du cordon ombilical (Quinn, 1993). D'autres auteurs ont démontré la présence d'ARNm de flk-1 dans les cellules endothéliales des sinusoïdes hépatiques adultes (Yamane, 1994), les glomérules adultes (Millauer, 1994) et les îlots β de Langerhans (Oberg, 1994).

De même l'inoculation concomitante de cellules tumorales et de cellules infectées par des récepteurs flk-1 dominants négatifs inhibe la croissance tumorale (Millauer, 1994).

L'un des buts de l'invention est de proposer l'utilisation d'anticorps anti-idiotypes de facteurs de croissance à affinité pour l'héparine permettant de cibler spécifiquement sur l'un ou l'autre de leurs récepteurs un nouveau type d'agonistes circulants.

L'un des autres aspects de l'invention est de fournir des agonistes de récepteurs de facteurs de croissance ayant une spécificité appropriée et une longue durée de demi-vie.

L'un des autres buts de l'invention est de fournir des images internes des domaines de liaison du VEGF à KDR qui du fait de leur structure immunoglobulinique sont circulants.

L'invention concerne des anticorps anti-idiotypiques du facteur de croissance endothéliale vasculaire pour la préparation d'un médicament destiné au traitement de pathologies impliquant des cellules endothéliales engagées dans un processus d'angiogénèse, soit pour inhiber l'angiogénèse, soit pour favoriser l'angiogénèse, sans affecter les cellules endothéliales quiescentes, ou pour la préparation d'un produit de diagnostic de pathologies impliquant des cellules endothéliales engagées dans un processus d'angiogénèse.

L'expression "cellules endothéliales engagées dans un processus d'angiogénèse" signifie cellules endothéliales migrant à travers la lame basale et se multipliant.

Pour déterminer si des cellules sont engagées dans un processus d'angiogénèse, on peut avoir recours à l'immunomarquage à l'aide d'anticorps dirigés contre l'intégrine β3 (Brooks et al., Cell, 1994, 79:1157-1164).

L'expression "cellules endothéliales quiescentes" signifie cellules endothéliales des vaisseaux adultes normaux, non angiogéniques.

L'invention concerne l'utilisation d'anticorps anti-idiotypiques du facteur de croissance endothéliale vasculaire pour la préparation d'un médicament destiné au traitement de pathologies impliquant les cellules endothéliales angiogéniques, par stimulation sélective du récepteur KDR.

L'expression "cellules endothéliales angiogéniques" désigne les cellules impliquées dans un processus d'angiogénèse.

Les anticorps anti-idiotypes de l'invention reconnaissent le récepteur humain KDR (ou le récepteur murin flk-1), mais ne reconnaissent pas le récepteur flt-1.

Grâce aux anticorps anti-idiotypiques de l'invention, on a pu mettre en évidence le fait que KDR (resp. flk-1) est la cible de l'angiogénèse pathologique.

Selon un mode de réalisation avantageux, l'invention concerne l'utilisation d'anticorps anti-idiotypiques du facteur de croissance endothéliale vasculaire pour la préparation d'un médicament destiné au traitement de pathologies impliquant des cellules endothéliales engagées dans un processus d'angiogénèse, pour inhiber l'angiogénèse, sans affecter les cellules endothéliales quiescentes, lequel anticorps anti-idiotypique est couplé à une toxine dont la fonction est de bloquèr la traduction des protéines.

Comme toxine, on peut citer la saporine, la ricine ou bien un élément radioactif tel que l'iode 125 ou 131.

Selon un autre mode de réalisation avantageux, l'invention concerne l'utilisation d'anticorps anti-idiotypiques du facteur de croissance endothéliale vasculaire selon pour 1a préparation d'un médicament destiné au traitement de pathologies impliquant des cellules endothéliales engagées dans un processus d'angiogénèse, pour inhiber l'angiogénèse, sans affecter les cellules endothéliales quiescentes, lequel anticorps anti-idiotypique est sous forme de son fragment Fab.

Comme pathologies dont le traitement nécessite l'inhibition de l'angiogénèse, on peut citer le cancer, les rétinopathies diabétiques et le rejet de greffes de cornée.

L'invention concerne l'utilisation d'anticorps anti-idiotypiques du facteur de croissance endothéliale vasculaire, pour la préparation d'un médicament destiné au traitement de pathologies impliquant des cellules endothéliales engagées dans un processus d'angiogénèse pour favoriser l'angiogénèse, sans affecter les cellules endothéliales quiescentes.

Comme pathologies dont le traitement nécessite la stimulation de l'angiogénèse, on peut citer la cicatrisation, la reperfusion de territoires ischémiés lors de thrombose artérielle ou veineuse.

L'invention concerne l'utilisation d'anticorps anti-idiotypiques du facteur de croissance endothéliale vasculaire pour la préparation d'un médicament destiné à stimuler l'angiogénèse physiologique pour augmenter la vitesse de la formation des vaisseaux sanguins au cours de la cicatrisation, de la maturation du corps jaune de l'ovaire ou stimuler l'angiogénèse au cours de pathologies obstructives des vaisseaux afin de reperfuser des territoires ischémiés lors de thrombose vasculaire.

Les anticorps anti-idiotypes de l'invention peuvent également être utilisés pour la préparation d'un produit de diagnostic de pathologies impliquant des cellules endothéliales engagées dans un processus d'angiogénèse.

La mise en évidence de l'absence de réaction des tissus sains à la stimulation de KDR par voie générale ouvre la voie à la cartographie de territoires exprimant le KDR ou le flk-1. Une injection de d'anticorps anti-idiotypiques iodés du facteur de croissance endothéliale vasculaire peut permettre de visualiser des tumeurs sans liaison à des organes sains.

L'invention concerne un anticorps anti-idiotypique du facteur de croissance endothéliale vasculaire caractérisé en ce qu'il est un ligand du récepteur humain KDR ou du récepteur murin flk-1 et qu'il n'est pas un ligand de flt.

L'invention concerne des anticorps anti-idiotypiques du facteur de croissance endothéliale vasculaire caractérisés en ce qu'ils présentent les propriétés suivantes :
- ils sont spécifiques vis-à-vis de KDR,
- ils sont circulants,
- ils présentent une durée de demi-vie d'environ 23 jours, notamment d'environ 21 jours, et en particulier de 22,5 jours,
- ils induisent la phosphorylation sur une tyrosine d'une protéine de 200 kDa,
- ils induisent la prolifération des cellules endothéliales vasculaires,
- ils n'induisent pas la migration de cellules endothéliales,
- ils stimulent l'angiogénèse,
- ils ne provoquent pas d'hypotension artérielle,
- ils n'affectent pas la perméabilité des vaisseaux.

La spécificité vis-à-vis de KDR peut être déterminée selon le test de compétition avec le VEGF radioiodé vis-à-vis de sa liaison à des cellules COS transfectées avec des vecteurs d'expression eucaryote contenant la séquence du KDR (Terman et al., Biochem. Biophys. Res. Commun., 1992, 187:1579-1586).

A la différence des anticorps anti-idiotypiques de l'invention, VEGF n'est pas spécifique vis-à-vis de KDR puisqu'il peut se lier au récepteur flt.

L'intérêt de la spécificité des anticorps anti-idiotypes de l'invention est de cibler sur les cellules endothéliales angiogéniques des drogues qui n'affectent pas les cellules endothéliales quiescentes.

L'expression "circulant" signifie véhiculé librement dans le sang circulant et non capté par les parois vasculaires.

A la différence des anticorps anti-idiotypes de l'invention, VEGF n'est pas circulant. L'intérêt de ce que les anticorps anti-idiotypes de l'invention soient circulants est de cibler sur les cellules endothéliales angiogéniques des drogues qui n'affectent pas les cellules endothéliales quiescentes.

S'agissant de la durée de demi-vie des anticorps anti-idiotypiques, elle est variable d'une espèce à l'autre ; par exemple pour le rat, elle est de 7 jours.

La durée de demi-vie des anticorps de l'invention peut être mesurée selon le test suivant : injection intraveineuse de 1 µCi de ligand radioiodé, puis recueil à différents intervalles de temps de sang et comptage de la radioactivité. La durée de demi-vie correspond au temps nécessaire pour que 50% de la radioactivité initiale ait disparu du sang circulant.

La durée de demi-vie du VEGF est inférieure à 6 minutes (Beuters et al., Circulation, 1995); la durée de demi-vie des IgG est de l'ordre de 23 jours.

La protéine de 200 kDa sur laquelle les anticorps-anti-idiotypiques induisent la phosphorylation d'une tyrosine est KDR.

Cet aspect signifie que l'activation de KDR par les anticorps anti-idiotypes peut déclencher des fonctions nécessitant la phosphorylation de KDR.

Cet aspect peut être mesuré par le test de phosphorylation décrit dans les exemples.

L'induction de la prolifération des cellules endothéliales vasculaires signifie qu'elles se multiplient.

Ceci peut être déterminé selon le test décrit dans les exemples.

Les anticorps de l'invention induisent la prolifération des cellules endothéliales vasculaires plus fortement que VEGF, c'est-à-dire dans un rapport d'environ 2 fois.

L'intérêt de cette augmentation de l'induction de la prolifération des cellules endothéliales par les anticorps anti-idiotypiques de l'invention est que la liaison des anticorps anti-idiotypes au récepteur KDR suivie de la phosphorylation sur une tyrosine de KDR suffit à déclencher la prolifération cellulaire.

L'absence de migration des cellules endothéliales signifie que les anticorps anti-idiotypes miment, parmi les effets déclenchés par le VEGF, ceux qui sont médiés par KDR et non ceux qui sont médiés par flt-1.

Cet aspect peut être mesuré par le test décrit dans les exemples.

A la différence des anticorps de l'invention, VEGF induit la migration des cellules endothéliales.

L'intérêt présenté par les anticorps de l'invention est de démontrer leur absence d'effet sur le récepteur flt-1 grâce aux tests de migration et de perméabilité explorant la fonction de flt-1.

La stimulation de l'angiogénèse signifie que la liaison des anticorps anti-idiotypes au récepteur KDR suivie de la phosphorylation sur une tyrosine de KDR et de la prolifération cellulaire suffisent à déclencher l'angiogénèse.

Ceci peut être quantifié par le test décrit dans les exemples.

Le fait que les anticorps de l'invention n'affectent pas la perméabilité des vaisseaux signifie que les anticorps anti-idiotypes miment, parmi les effets déclenchés par le VEGF, ceux qui sont médiés par KDR et non ceux qui sont médiés par flt-1, par exemple la perméabilité vasculaire.

Ceci peut être déterminé selon le test suivant : des cellules endothéliales de cornée sont ensemencées (40 000 cellules/cm²) sur des filtres (12 mm de diamètre) préalablement incubées 16 heures à 4°C avec du PBS supplémenté de 2 mg/ml de gélatine (Millicell-Millipore). Les puits sont disposés dans des boîtes de 24 puits (diamètre 16 mm). Après 5 jours, la résistance électrique entre le compartiment intracellulaire et extracellulaire est mesurée (de l'ordre de 180 Ω). Les modulateurs sont ajoutés et la résistance mesurée toutes les 10 minutes. Sous l'effet de VEGF (5-50 ng/ml) la résistance chute à 150 m en 90 minutes environ. Une diminution de la résistance traduit donc une augmentation de la perméabilité. Cette fonction est médiée par le récepteur flt-1 et non le récepteur KDR.

A la différence des anticorps de l'invention, VEGF affecte la perméabilité des vaisseaux. L'intérêt de l'invention est donc de montrer que les anticorps anti-idiotypes ne stimulent pas *in vivo* les effets médiés par le récepteur flt-1.

L'invention concerne également le fragment Fab des anticorps anti-idiotypiques selon l'invention.

L'invention concerne également le complexe entre un anticorps anti-idiotypique selon l'invention et une toxine, en particulier choisie parmi la saporine, la ricine ou bien entre un anticorps anti-idiotype selon l'invention et un élément radioactif tel que l'iode 125 ou 131.

L'invention a également pour objet un anticorps anti-idiotypique selon l'invention, susceptible d'être obtenu selon le procédé suivant :
- on injecte du VEGF purifié chez un animal, notamment un lapin,
- on prélève le sang pour récupérer les Ig purifiées contenant des IgG anti-VEGF spécifiques, par exemple par chromatographie d'affinité pour la protéine A, puis dans une étape éventuelle, on purifie les IgG anti-VEGF spécifiques à partir des Ig purifiées, par exemple par chromatographie d'affinité pour le VEGF,
- on injecte les susdites Ig purifiées ou les susdites IgG anti-VEGF purifiées chez l'animal de la même espèce que celui utilisé lors de l'injection de VEGF, notamment dans les ganglions poplités de lapin de même origine que celui utilisé lors de l'injection de VEGF,
- on prélève le sang pour récupérer les Ig totales, par exemple par la protéine A, puis pour soumettre les Ig totales à deux immunoadsorbtions :
   . une immunoadsorbtion sur une colonne d'affinité préparée avec les Ig pré-immunes du lapin qui a servi à faire les IgG anti-VEGF, pour éliminer les anticorps anti-allotypes ou isotypes,
   . une immunoadsorbtion sur une colonne d'affinité préparée avec des IgG anti-VEGF, pour purifier les anti-idiotypes.

Pour faire la preuve de l'action *in vivo* des anti-idiotypes de l'invention, seule la purification sur protéine A sépharose est nécessaire.

L'invention concerne également un procédé de préparation d'un anticorps anti-idiotypique selon l'invention, caractérisé en ce que :
- on injecte du VEGF purifié chez un animal, notamment un lapin,
- on prélève le sang pour récupérer les Ig purifiées contenant des IgG anti-VEGF spécifiques, par exemple par chromatographie d'affinité pour la protéine A, puis dans une étape éventuelle, on purifie les IgG anti-VEGF spécifiques à partir des Ig purifiées, par exemple par chromatographie d'affinité pour le VEGF,
- on injecte les susdites Ig purifiées ou les susdites IgG anti-VEGF purifiées chez l'animal de la même espèce que celui utilisé lors de l'injection de VEGF, notamment dans les ganglions poplités de lapin de la même origine que celui utilisé lors de l'injection de VEGF,
- on prélève le sang pour récupérer les Ig totales, par exemple par la protéine A, puis pour soumettre les Ig totales à deux immunoadsorbtions :
   . une immunoadsorbtion sur une colonne d'affinité préparée avec les Ig pré-immunes du lapin qui a servi à faire les IgG anti-VEGF, pour éliminer les anticorps anti-allotypes ou isotypes,
   . une immunoadsorbtion sur une colonne d'affinité préparée avec des IgG anti-VEGF, pour purifier les anti-idiotypes.

Pour préparer les fragments Fab des anticorps de l'invention, on peut procéder de la façon suivante : les fragment Fab sont préparés à partir de 200 mg d'Ig2 Id dissoutes dans 42 ml contenant 3,5 mg de papaine et 0,1 M EDTA (ethylene diamine tetra acetic acid) et 0,1 M de cystéine. Le mélange est incubé 4 heures à 37°C, puis la réaction est arrêtée par 4,6 ml de iodacétamide 0,3 M. Le mélange est alors dialysé contre 2 litres de PBS à 4°C pendant 16 heures, puis chromatographié sur une colonne de 10 ml de protéine A sépharose. Les fragments Fab sont récupérés dans le volume mort.

L'invention est également relative à des compositions pharmaceutiques caractérisées en ce qu'elles comprennent à titre de substance active au moins un anti-idiotype selon l'invention, ou au moins un fragment Fab selon l'invention ou au moins un complexe selon l'invention.

L'invention est également relative à des compositions pharmaceutiques déduites des séquences des domaines hypervariables des fragments Fab des anticorps anti-idiotypes.

L'invention est également relative à des compositions pharmaceutiques déduites de la structure tridimensionnelle des fragments Fab des anticorps anti-idiotypes déterminée par cristallographie et diffraction aux rayons X, dichroïsme circulaire, résonnance magnétique nucléaire.

### DESCRIPTION DES FIGURES :

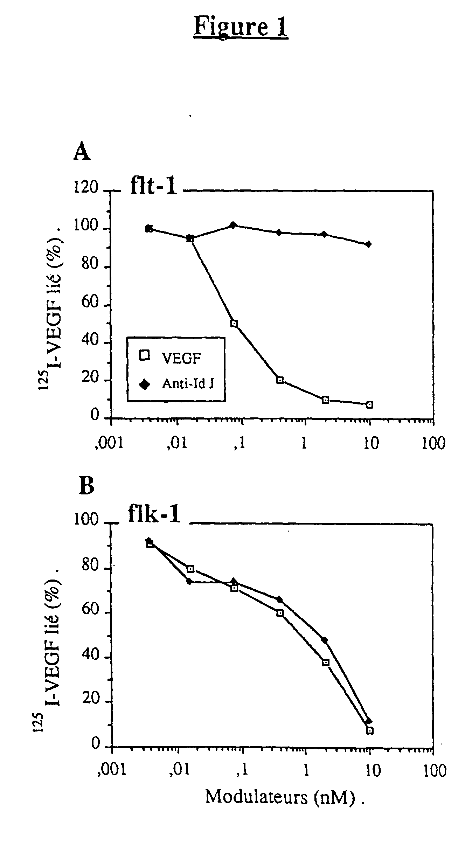

La Figure 1 représente l'inhibition de la liaison du VEGF iodé à ses récepteurs par l'anticorps anti-idiotypique J. On en conclut que l'anticorps J ne déplace pas la liaison de VEGF au récepteur flt-1.

Les concentrations indiquées de VEGF ou anti-Id J sont incubées avec des cellules COS transfectées 48 heures au préalable par 1 µg/ml de vecteur pSV7d contenant la séquence de flt-1 (partie A de la Figure) ou de flk-1 (partie B de la Figure) en présence de 1 ng/ml (partie A de la Figure) ou de 10 ng/ml (partie B de la Figure) de VEGF iodé à 4°C. Après 3 heures les cellules sont rincées, lysées et la radioactivité est comptée dans un compteur gamma.

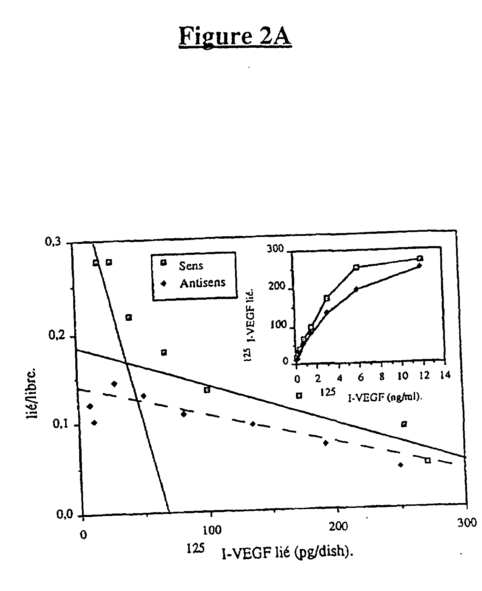

La figure 2A représente l'inhibition de la liaison du VEGF à son récepteur de haute affinité par la transfection de cellules FBAE par des oligonucléotides antisens de KDR

Des cellules FBAE subconfluentes ensemencées dans des plaques de 12 puits sont transfectées avec 2 µM d'oligonucléotides sens ( 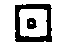 ) ou antisens (◆) de KDR. 24 heures après les cellules sont incubées pendant trois heures à 4°C en présence de concentrations croissantes de VEGF iodé dans un volume final de 500 µl. On en conclut que J ne se lie pas aux cellules n'exprimant pas flk-1.

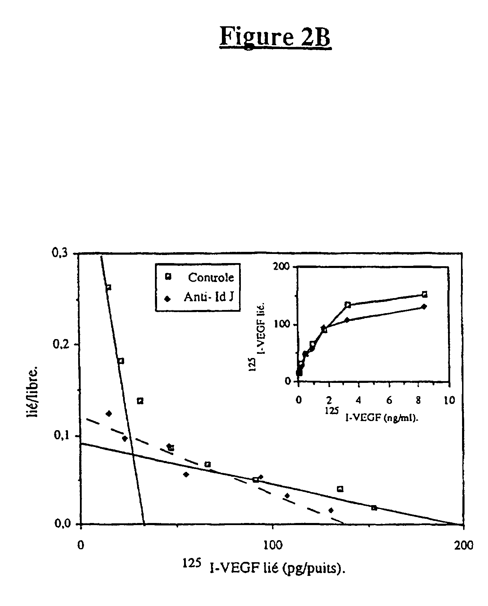

La Figure 2B représente l'inhibition de la liaison du VEGF à son récepteur de haute affinité sur des cellules FBAE par une pré-incubation avec l'anti-idiotype J (anti-Id J).

Des cellules FBAE subconfluentes sont ensemencées dans des puits de 10 cm² et incubées pour une internalisation avec 5 µg/ml d'anti-Id J ou d'IgG pré-immunes à 37°C pendant 90 mn. Les plaques sont ensuite transférées à 4°C et incubées avec des concentrations variables de VEGF iodé. La liaison non spécifique est déterminée en présence de 1 µg/ml de VEGF froid (encart). Les données sont ensuite exprimées selon la représentation de Scatchard. On en conclut que J se lie à flk-1 et seulement à flk-1.

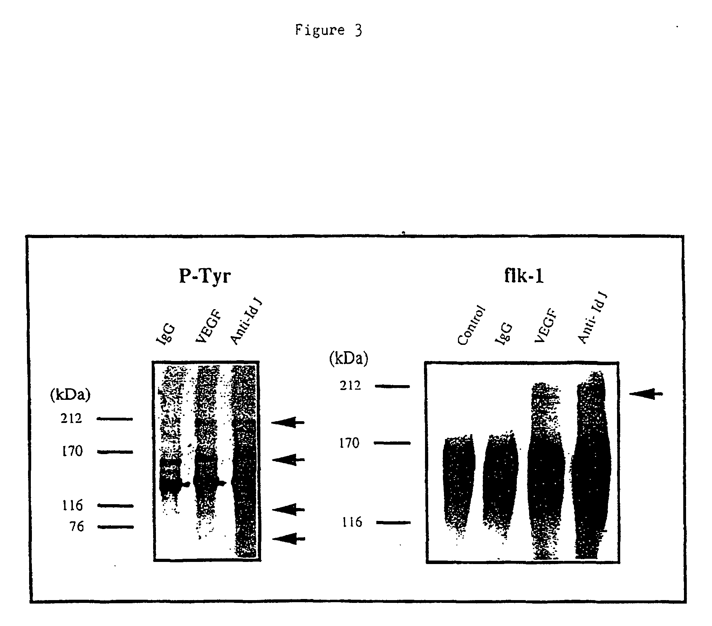

La Figure 3 représente l'induction de la phosphorylation par l'anti-idiotype J sur des résidus tyrosine de KDR/flk-1 dans des cellules FBAE.

Des cellules FBAE sont stimulées 10 mn à 37°C par des IgG pré-immunes, du VEGF ou de l'anti-Id J, puis elles sont lysées en tampon RIPA et le lysat est immunoprécipité avec un anticorps monoclonal anti-phosphotyrosine (PY22). Les immuns complexes sont collectés sur des billes de protéine A sépharose, soumis à une électrophorèse en gel de polyacrylamide, transférés sur un filtre de nitrocellulose et révélés soit par un anticorps monoclonal anti-phosphotyrosine PY22, soit par un anticorps polyclonal anti-flk-1.

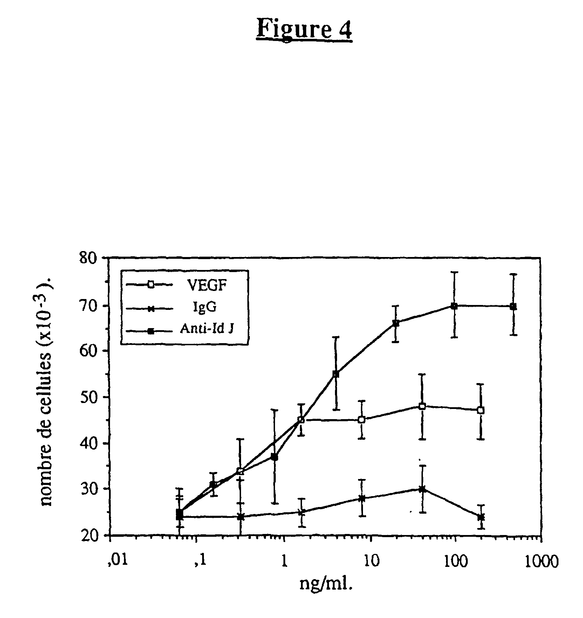

La Figure 4 représente la stimulation de la prolifération des cellules FBAE par l'anti-idiotype J. On en conclut que J phosphoryle le récepteur flk-1 comme le fait le VEGF.

Des cellules FBAE sont ensemencées à 5000 cellules par puits dans des plaques de 12 puits en présence de VEGF, anti-Id J ou d'IgG pré-immunes à des concentrations variables allant de 0,01 ng/ml à 1000 ng/ml. Après cinq jours, les cellules sont trypsinisées et comptées. Les données représentées sont les moyennes de trois points et les expériences ont été réalisées quatre fois avec des résultats similaires. On en conclut que J stimule la prolifération des cellules FBAE.

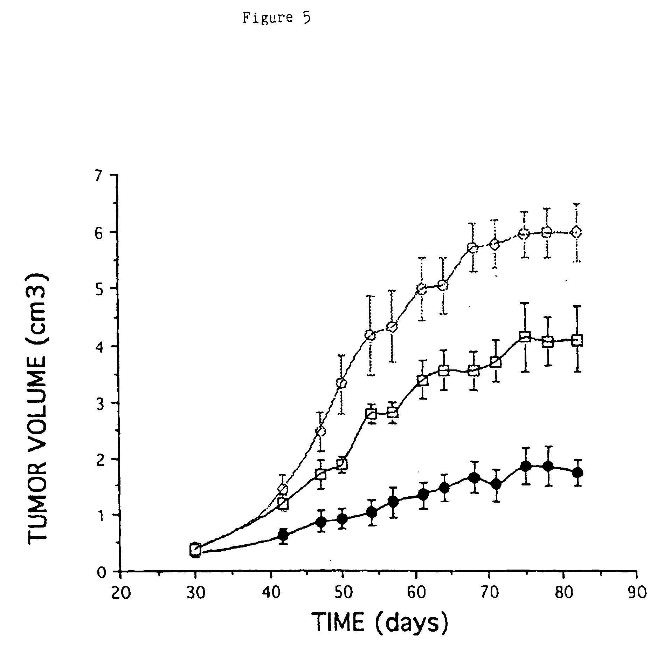

La Figure 5 représente les effets de l'injection de Ig2 Id sur le volume tumoral.

Des fragments d'adénocarcinome prostatique hormono-indépendant correspondant à un score IX de Gleason (3x3x3 mm) ont été greffés à des souris nu/nu femelles. Un mois plus tard, quand elles deviennent cliniquement palpables, les animaux (n=8-10 pour chaque groupe) reçoivent 2 fois par semaine 200 µg d'Ig1 T neutralisant l'activité du VEGF (V-IgG), ou d'Ig1 PI (PI-IgG) ou d'Ig2 Id (J-IgG). Le volume tumoral a été mesuré 2 fois par semaine à l'aide d'un pied à coulisses et les souris ont été pesées. Les animaux ont été sacrifiés 3 mois après et les tumeurs photographiées.

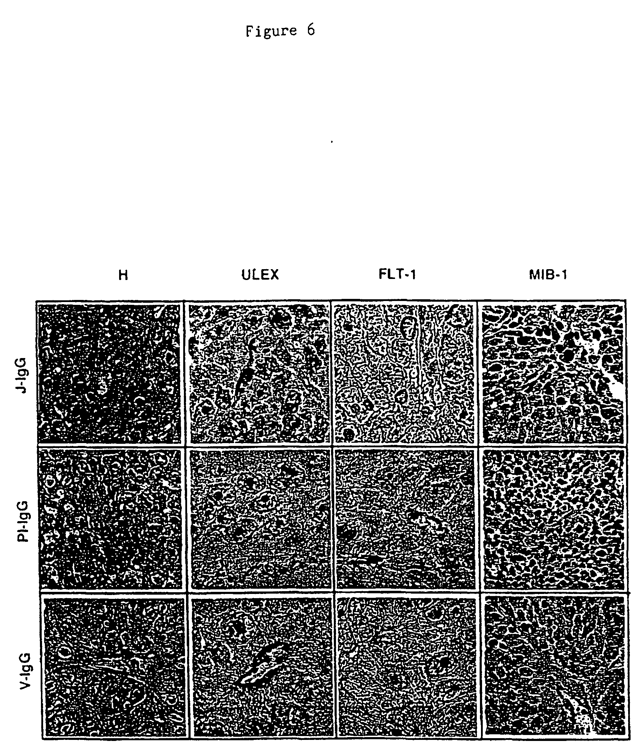

La Figure 6 représente l'analyse histologique (a-c), le marquage de la vascularisation (d-i) et de la prolifération (j-1) de xénogreffes d'adénocarcinome traitées par V-IgG, PI-IgG ou J-IgG.

Les animaux traités comme indiqué dans la légende de la figure 5 ont été sacrifiés 3 mois après l'implantation des xénogreffes, puis des coupes sont pratiquées dans ces tumeurs et colorées avec l'hématoxyline-éosine (a-c) Des coupes adjacentes ont été révélées avec le marqueur ulex (d-f) (Ulex Europeaus conjugué à la peroxydase Sigma, réf. L8146), l'anticorps anti-flt-1 (Santa Cruz) (g-i) ou mib-1 (j-1). On en conclut que J stimule le nombre de vaisseaux et la prolifération des cellules cancéreuses.

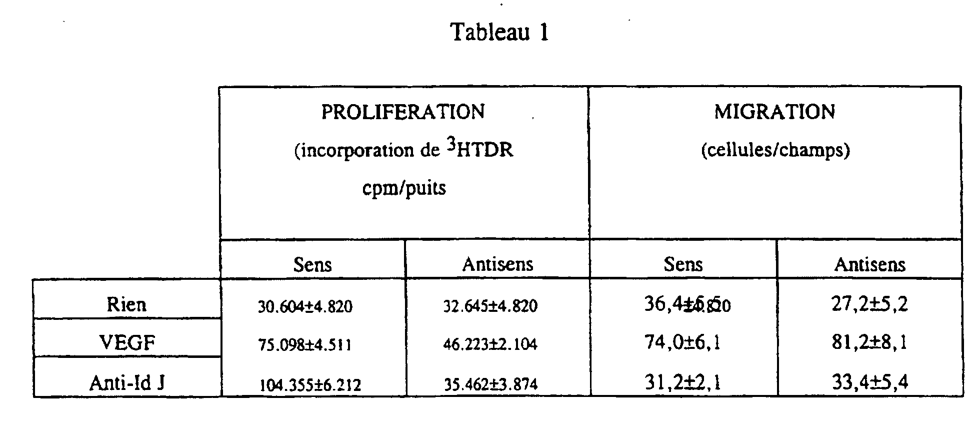

Le tableau 1 représente les effets biologiques du VEGF et de l'anti-Id J sur des cellules FBAE transfectées par des oligonucléotides sens ou antisens de KDR.

Des cellules FBAE subconfluentes sont transfectées par des oligonucléotides sens ou antisens de KDR comme décrit dans la description ci-dessus. Vingt-quatre heures après, les cellules sont transférées en milieu sans sérum, elles sont ensuite incubées en présence de 50 pM de VEGF, l'anti-Id J ou rien, puis elles sont marquées 4 heures avec de la thymidine tritiée après 20 heures de stimulation. Parallèlement, des expériences de migration sont réalisées sur des cellules confluentes. Une incision en forme de croix de Malte est effectuée sur la monocouche cellulaire. Les puits sont lavés avec du milieu sans sérum et les cellules sont incubées avec des concentrations de modulateurs dix fois plus élevées. Vingt-quatre heures après, les cellules sont colorées au May-Grumwald-Giemsa et les cellules ayant migré sont comptées sur huit champs, chaque point est réalisé en triple. Ces expériences ont été faites trois fois avec des résultats similaires. On en conclut que J ne mime qu'une partie des effets du VEGF : il stimule la prolifération mais pas la migration.

### ABREVIATIONS UTILISEES DANS LES EXEMPLES :

PBS (phosphate buffer saline) : solution saline de tampon phosphate.
Ig1PI : immunoglobulines de lapin purifiées par protéine A-sépharose à partir de sang prélevé avant l'immunisation par le VEGF.
Ig1T : immunoglobulines de lapin purifiées par protéine A-sépharose à partir de sang prélevé après immunisation par le VEGF.
Ig1 anti-VEGF : Ig1T purifiées par VEGF-sépharose.
Ig2 Id : immunoglobulines de lapin 2 purifiées par protéine A- sépharose à partir de sang prélevé après l'immunisation par le Ig1T.
Ig2J (également désigné par J) : Ig2 Id purifiées par Ig1 PI-sépharose puis Ig1 anti-VEGF sépharose.

### METHODES D'ETUDE

### 1. Fabrication d'anticorps anti-idiotypes de VEGF165.

- Obtention des IgG pré-immunes (Ig1 PI) :

Avant chaque immunisation, du sang est prélevé, le sérum est fractionné immédiatement après le recueil et 15 ml de sérum sont chromatographiés sur une colonne de protéine A (0,9x18 cm). La colonne est lavée par du PBS et les immunoglobulines sont éluées par de la glycine 0,2 M tamponnée à pH 2,5, immédiatement neutralisées par adjonction de 1/5 du volume de K2HPO4 1 M, puis dialysées contre du PBS. Les immunoglobulines (Ig1 PI) sont conservées à 80°C jusqu'à leur utilisation.

### 1.1. Obtention des anticorps anti-idiotypiques murins.

Des cellules AtT20 cultivées jusqu'à la confluence puis transférées dans du milieu DMEM (Dulbecco's Modified Eagle Medium (Gibco, réf. 13016 027) contenant 5 µg/ml d'insuline et 10 µg/ml de transferrine sont stimulées par 5 ng/ml de FGF2 recombinant produit chez *Escherichia coli* (don du Dr. Hervé Prats, U397 INSERM, Toulouse) pendant 48 heures pour augmenter la production de VEGF (Plouët, 1992).

Le milieu conditionné (30 litres) a été purifié comme décrit précédemment (Plouët, 1989). Quarante µg de VEGF murin sont séparés par électrophorèse sur gel de polyacrylamide en condition dénaturante, transférés sur une feuille de nitrocellulose.

La fraction contenant le VEGF a été découpée puis dissoute dans 1 ml de diméthylsulfoxyde. Dix µg de VEGF sont émulsionnés dans 0.25 ml d'adjuvant de Freund complet puis injectés chez un lapin "Fauve de Bourgogne" (Iffa-Credo), 4 fois à 15 jours d'intervalle.

Le sang prélevé entre 3 et 7 mois après la première injection est fractionné et les Ig sont purifiées par chromatographie d'affinité pour la protéine A; ces Ig purifiées sont appelées Ig1T.

Des lapins "Fauve de Bourgogne" anesthésiés reçoivent une injection de 0,5 ml de PBS contenant du bleu Evans dans le coussinet plantaire afin de colorer le système lymphatique. Une incision cutanée est alors pratiquée, les plans cutanés libérés et le ganglion devenu bleu est alors visualisé.

Dix µg d'Ig1T (5 µl) émulsionnées dans 100 µl d'un mélange 1-1 de PBS-adjuvant de Freund complet sont injectés dans le ganglion poplité de chacun des lapins 30 minutes après.

L'injection d'adjuvant crée un granulome inflammatoire qui permet de palper le ganglion et donc de pratiquer ultérieurement des injections transcutanées sans qu'il soit nécessaire d'inciser le plan cutané. 3, 6 et 9 semaines après les lapins reçoivent une injection intra-ganglionnaire de 10 µg d'Ig1T.

Le sang prélevé entre 4 et 7 mois après la première injection est purifié comme décrit précédemment. Les Ig anti-idiotypes (Ig2J) sont alors purifiées par une série de 2 chromatographies d'affinité, comme indiqué dans les paragraphes 2 et 3 ci-après.

### 1.2. Obtention des anticorps anti-idiotypiques humains

Le VEGF 165 a été produit dans des cellules CHO transfectées de façon stable par un plasmide d'expression (prCEN-1, F. Bayard, Unité 397 INSERM) contenant l'ADNc du VEGF 165 (don de Judith Abraham, Scios Nova, Mountain View, CA).

Les cellules sont cultivées à confluence en milieu DMEM contenant les antibiotiques et 10% de sérum de veau nouveau-né, puis rincées 3 fois avec du milieu DMEM sans sérum et incubées 48 heures dans du milieu DMEM contenant 5 µg/ml d'insuline et 10 µg/ml de transferrine.

Deux jours après, le milieu est collecté, centrifugé (20000g, 30 minutes) puis chromatographié sur héparine sépharose (0,9 x 6 cm) préalablement équilibrée dans du tampon Tris 0,01 M, pH 7,2 contenant 0,05 M NaCl. Après le lavage de la colonne dans le même tampon, le VEGF est élué par un gradient de concentration de NaCl dans le tampon Tris. L'activité biologique du VEGF est mesurée par son activité mitogène (Plouët, 1989). Les milieux sont alors centrifugés et la quantité de VEGF produite quantifiée par radiorécepteuressai et par la mesure de compétition avec le VEGF iodé vis-à-vis de la liaison à ses récepteurs.

Dix µg de VEGF sont injectés dans les ganglions poplités de lapin New-Zealand (INRA, Toulouse) comme décrit plus haut. Après 3-7 mois les Ig1T sont examinées pour leur capacité à neutraliser l'effet mitogène du VEGF sur les cellules ACE (cellules endothéliales de capillaires de cortex de glandes surrénales bovines).

Des lapins co-isogéniques reçoivent des injections de 10 µg d'Ig1T selon le protocole décrit précédemment.

### 2- Construction des immunoadsorbants

### VEGF-sépharose

Un g de sépharose-CNBr (Pharmacia) est mis en présence avec 2 mg de VEGF préalablement dialysé contre du tampon carbonate 0,1 M pH 8,5 pendant une nuit. Après ce temps la matrice est lavée par centrifugations (2000 g, 5 minutes) 3 fois avec 10 ml de tampon carbonate. Les fonctions amines restées libres sont ensuite bloquées par incubation avec 5 ml d'éthanolamine 0,2 M. La matrice est relavée 3 fois avec du PBS, une fois avec de la glycine 0,2 M pH 2,5 pendant 5 minutes, puis avec 10 ml de K2HPO4. La matrice est alors conservée dans du PBS supplémenté de 0,02% d'azoture de sodium jusqu'à utilisation.

### Ig1 pré-immunes

La matrice d'affinité est préparée comme précédemment en mettant en contact 1 g de CNBr sépharose et 5 mg de Ig1PI.

### Ig1 anti-VEGF

Cinquante mg d'Ig1T sont chromatographiées sur la colonne de sépharose-VEGF, éluées par de la glycine pH 2,5 0,2 M, dialysées et congelées à -80 °C jusqu'à leur utilisation. Les Ig spécifiques de VEGF représentent 8 % des Ig1T.

### 3- Purification des Ig anti-idiotypiques.

Les Ig anti-idiotypiques purifiées sur protéine A sépharose (Ig2 Id) sont purifiées par une séquence de 2 chromatographies d'affinité : chromatographie sur 1g1PI-sépharose et une chromatographie sur Ig1 anti-VEGF-sépharose.

### Ig1 PI-sépharose

Cinquante mg d'Ig2 Id sont inoculées sur la colonne d'Ig1PI-sépharose disposée dans une colonne Economo-pack (0,2x1 cm). Une fois que les Ig2 ont été séparées des éventuelles Ig2 Id dirigées contre les isotypes et les allotypes de Ig1 PI, donc liées au Ig1 PI sépharose, la fraction non retenue, donc composée des Ig régulières non immunes et des Ig2 J dirigées contre les domaines des Ig1 reconnaissant le VEGF, est déposée sur une colonne d'Ig1 anti-VEGF sépharose.

On n'a jamais noté que cette procédure permettait de séparer une quantité mesurable d'Ig anti-isotypes et anti-allotypes.

### Ig1 anti-VEGF-sépharose

Les Ig anti-id non retenues sur la colonne d'Ig1PI, donc déplétées des éventuels anticorps anti-isotypes et anti-allotypes, sont inoculées sur la colonne d'Ig1 VEGF-sépharose disposée dans une colonne Economo-pack (0,2x1 cm).

La colonne est ensuite lavée avec 10 ml de PBS, puis les Ig retenues sont éluées avec de la glycine 0,2 M pH 2,5, immédiatement neutralisées avec un cinquième du volume avec K2HPO4 1 M, puis dialysées contre du PBS.

Cette procédure permet de purifier des immunoglobulines anti-idiotypiques (Ig2J) qui représentent 2-3 % des Ig2 Id totales.

### 4- Etude de la spécificité des anticorps

Le criblage des anticorps anti-idiotypes s'effectue selon la séquence suivante mise au point au laboratoire:

Des cellules COS ensemencées à 30 000 cellules par puits de 2 cm² sont cultivées en milieu DMEM contenant 10% de sérum de veau foetal, 100 UI/ml de pénicilline et 50 µg/ml de streptomycine. Deux jours après les cellules sont transférées en milieu ne contenant ni sérum ni antibiotiques et incubées avec 2 µg/ml de plasmide pSV7d contenant l'ADNc de flt-1 ou de flk-1 (dons de L. Williams, UCSF) et 10 µg/ml de lipofectine pendant 30 minutes.

Après 6 heures, le milieu est remplacé par du DMEM contenant 10% de sérum de veau foetal pendant 48 heures.

La liaison de VEGF radioiodé (1-2 10⁵ cpm/ng) aux cellules COS transfectées est mesurée à 4 °C. Les cellules sont lavées 2 fois avec du tampon de liaison (DMEM contenant 20 mM Hepes et 1 mg/ml de gélatine ajustée à un pH de 7.4. Les concentrations désirées de VEGF iodé (1 ng/ml pour les cellules COS/flt-1 et 10 ng/ml pour les cellules COS/flk-1) sont ajoutées avec des concentrations variables de VEGF non marqué ou d'Ig2 Id ou Ig2 J sous un volume final de 0.5 ml. La liaison non spécifique est déterminée en présence d'un excès -500 ng- de VEGF purifié. Les liaisons totale et non spécifique sont déterminées en double.

Après deux heures les cellules sont lavées 3 fois avec du tampon froid. et lysées avec 0.5 ml de NaOH 0.2 M. L'iode 125 contenu dans le matériel solubilisé est compté dans un compteur gamma.

En parallèle des cellules FBAE (foetal bovine aortic endothelial cells) ensemencées à 100 000 cellules par puits de 10 cm² sont cultivées en milieu DMEM contenant 10% de sérum de veau foetal et 100 UI/ml de pénicilline et 50 mg/ml de streptomycine. 2 jours après les cellules sont transférées en milieu ne contenant ni sérum ni antibiotiques et incubées avec 10 mg/ml d'oligonucléotides sens ou antisens du KDR préalablement incubés avec 10 mg/ml de lipofectine pendant 30 minutes.

Après 6 heures, le milieu est remplacé par du DMEM contenant 10% de sérum de veau foetal. pendant 24 heures. Des cellules FBAE subconfluentes non transfectées sont aussi incubées 90 minutes avec les IgG anti-idiotypiques ou les Ig1 PI. Les cellules sont alors transférées à 4°C puis incubées 3 heures avec des concentrations variables de VEGF iodé en présence (liaison non spécifique) ou absence (liaison totale) de 1 µg/ml de VEGF. Les données sont alors analysées selon la représentation de Scatchard (Scatchard, 1949) par le programme de Munson (Munson, 1980).

### 5. Activités biologiques des anticorps

### Phosphorylation des récepteurs

Des cellules FBAE sont stimulées 10 mn à 37°C avec 1 nM de VEGF ou Ig2 J puis elles sont lysées en tampon RIPA (Tris 50 mM pH 7,5, Nonidet P 40 Sigma, réf. N6507, 1%, EDTA 1 mM, 5 µg/ml de chacun des inhibiteurs de protéases suivants : benzamidine, pepstatine, leupeptine, aprotinine et le lysat est immunoprécipité avec un anticorps monoclonal anti- phosphotyrosine (PY22, Amersham). Les immuns complexes sont collectés sur des billes de protéine A-sépharose puis séparés par une électrophorèse en gel de polyacrylamide. Les protéines sont transférées sur un filtre de nitrocellulose et révélées par soit l'anticorps monoclonal anti-phosphotyrosine PY22 (figure 3), soit par un anticorps polyclonal commercial (Santa-Cruz) anti-flk-1 (figure 3).

### Prolifération

Des cellules FBAE ensemencées à 20 000 cellules par puits de 4 cm² sont cultivées en milieu DMEM contenant 10% de sérum de veau foetal et 100 UI/ml de pénicilline et 50 µg/ml de streptomycine. Deux jours après les cellules sont transférées en milieu ne contenant ni sérum ni antibiotiques et incubées avec 10 µg/ml d'oligonucléotides sens ou antisens du KDR préalablement incubés avec 10 µg/ml de lipofectine pendant 30 minutes.

Après 6 heures, le milieu est remplacé par du DMEM contenant 10% de sérum de veau foetal. 24 heures après les cellules sont transférées en milieu sans sérum puis alors incubées en présence de 50 pM de VEGF, d'Ig2 J ou de PBS seul. Entre la 68ème et la 72ème heure 1 microcurie (µCi) de thymidine tritiée est ajouté. Les cellules sont rincées 3 fois avec du PBS à 4 °C, 1 fois avec de l'acide trichloracétique dilué à 10%, puis lysées dans NaOH 0.2 M. Le lysat est alors compté à l'aide d'un compteur à scintillation. .

Dans des expériences similaires, des cellules FBAE sont ensemencées à 5000 cellules par puits dans des plaques de 12 puits en présence de VEGF, Ig2 J ou Ig1 PI à des concentrations variables allant de 0,01 ng/ml à 1000 ng/ml. Après cinq jours, les cellules sont trypsinisées et comptées. Les données représentées sont les moyennes de trois points et les expériences ont été réalisées quatre fois avec des résultats similaires.

### Migration

Des cellules ACE sont ensemencées à 100 000 cellules par puits de 4 cm². Des cellules sont enlevées de la monocouche selon une aire représentant une croix de Malte à l'aide d'un cône de surface calibrée. Les cellules sont rincées et incubées avec des concentrations de modulateurs (VEGF, Ig2 J, PBS) dix fois plus élevées que celles utilisées pour les expériences de prolifération. 24 heures après les cellules sont colorées au May-Grumwald-Giemsa et les cellules ayant migré sont comptées sur huit champs, chaque point étant réalisé en triple. Ces expériences ont été faites trois fois avec des résultats similaires.

### Effet biologique sur la perméabilité :

Des cellules endothéliales (10⁵/cm²) de cornée sont déposées sur des filtres (diamètre 0,45 m, Transwell*, Costar) préalablement incubés avec 1 mg/ml de collagène type 1 dissous dans du PBS. Le filtre est alors déposé dans un puits de boîte de culture multipuits (Nunc) contenant 0.5 ml de milieu DMEM et 15% de sérum de veau foetal dans la chambre supérieure et la chambre inférieure. Trois jours après que les cellules aient atteint la confluence, les cellules sont rincées et incubées dans du milieu sans sérum. La résistance de la couche de cellules est mesurée (appareillage Millicel*, Millipore). Dans 3 séries de puits identiques (3 puits par condition), 20 ng/ml de VEGF ou de facteur de croissance du placenta (qui ne se lie qu'au récepteur flt-1) ou 200 ng d'anticorps Ig2 J immunopurifié sont incubés dans la chambre supérieure et la résistance est mesurée toutes les 10 minutes. La résistance diminue dès 15 minutes, est minimale à 30 minutes et augmente ensuite pour atteindre son niveau de base après 90 minutes uniquement dans les puits traités par VEGF ou le facteur de croissance du placenta, et pas dans ceux traités par Ig2 J.

Ces résultats montrent que, la résistance diminuant, la perméabilité augmente par un mécanisme secondaire à l'activation du récepteur flt-1. Ig2 J ne se liant pas à flt-1, il n'affecte effectivement pas la perméabilité.

### Hypotension :

Le VEGF injecté par voie intravasculaire déclenche une hypotension, pas les anticorps anti-idiotypes du VEGF (qui n'agissent que sur les cellules endothéliales ayant acquis un phénotype angiogénique).

### Angiogénèse cornéenne

Des lenticules imprégnés de 2 µl de véhicule (PBS contenant 50 mg de sérum albumine bovine) ou de véhicule et 200 ng de VEGF ou 600 ng d'Ig2 J sont insérés dans le stroma cornéen à 2 mm du limbe chez des lapins New-Zealand. Après 12 jours la surface néovascularisée en regard de l'implant est quantifiée sur une échelle de 0 à 4 (Favard, 1992).

### Angiogénèse tumorale

Un adénocarcinome prostatique (grade IX de Gleason) a été propagé sériellement chez des souris immunodéprimées (Iffa-Credo). Un mois après les souris ont reçu 2 fois par semaine une injection intrapéritonéale de 200 µg d'Ig1T ou IgG1 PI ou Ig2 Id (toutes Ig purifiées par affinité pour la protéine A sépharose). Les tumeurs ont été mesurées 2 fois par semaine. Les souris ont été sacrifiées 2 mois après. Les coupes de tumeurs ont été incubées avec des anticorps Mib-1 (Immunotech) qui ne marquent que les cellules en phase mitotique, la lectine ulex (European) conjuguée à la peroxydase (Sigma) qui ne marque que les cellules endothéliales; et l'anticorps anti-flt-1.

### RESULTATS

Aucun sérum pré-immun (n=18) ne contenait d'IgG interférant avec la liaison du VEGF iodé sur des cellules COS transfectées par des vecteurs d'expression des récepteurs flt-1 ou flk-1. En revanche 20% des sérums anti-idiotypiques contiennent des IgG se liant à flk-1. Une préparation d'Ig2 Id purifiées à partir de sérum recueilli entre 4 et 7 mois après l'immunisation a été sélectionnée (Ig2 J) qui inhibe totalement la liaison du VEGF iodé aux cellules COS transfectées avec flk-1 (la concentration qui déclenche 50% de l'inhibition est d'environ 400 ng) mais n'interfèrent pas avec sa liaison aux cellules transfectées par flt-1 (Figure 1).

La transfection de cellules FBAE par des oligonucléotides antisens de 15 mers centrés sur le codon d'initiation de la traduction de KDR inhibe 20-25 % des sites de liaison du VEGF iodé. L'analyse des résultats selon Scatchard a montré que cette réduction de la liaison s'effectuait exclusivement sur le site de liaison de plus haute affinité 5 pM (Figure 2A). La liaison à ce site est aussi inhibée par la pré-incubation de cellules FBAE par les Ig2 J (Figure 2B).

Ig2 J et VEGF induisent la phosphorylation sur une tyrosine d'une protéine de 200 kDa. La révélation de cette espèce moléculaire par des anticorps dirigés contre flk-1 atteste que le récepteur flk-1 est bien phosphorylé par Ig2 J (Figure 3).

Ig2 J et VEGF induisent la prolifération des cellules ACE (la moitié de la prolifération maximale est obtenue avec 1,2 ng de Ig2J et 0,5 ng de VEGF) à un niveau significativement supérieur à celui obtenu en présence de VEGF correspondant à 1,8 (VEGF) et 3,1 (Ig2J) fois le nombre de cellules comptées dans les puits non stimulés (Figure 4).

En revanche Ig2 J n'induisent pas de migration des cellules endothéliales (Table 1).

Afin de vérifier si ces Ig modulaient l'angiogénèse, des implants contenant 200 ng de VEGF ou 600 ng De Ig2 J ou le PBS seul ont été greffés dans le stroma cornéen de lapins. 12 jours après, il n'a pas été observé de néovascularisation en regard des implants contenant le PBS seul. En revanche une néovascularisation d'intensité équivalente a été observée en regard des implants contenant 5 pmoles de VEGF ou Ig2 J (2.78 et 2.45 respectivement). L'analyse histologique de la rétine n'a montré aucune réaction proliférative bien que le VEGF soit synthétisé, du moins *in vitro*, par les cellules endothéliales, les péricytes et épithéliales pigmentées.

### Exemple 1 :

### Tumeur de la prostate :

Le VEGF étant actuellement reconnu comme l'inducteur majeur de l'angiogénèse tumorale, ces Ig2 Id ont été injectées chez des souris greffées un mois auparavant des fragments d'adénocarcinome prostatique. Après 1 mois de traitement par les Ig2 J, le volume des tumeurs est significativement supérieur à celui des tumeurs traitées par des Ig1 PI (Figure 5).

L'analyse immunohistochimique des coupes de tumeur a montré que ces anticorps induisent *in vivo* une prolifération cellulaire mesurée par immunohistochimie avec l'anticorps mib-1. En revanche aucune cellule positive par dans les organes sains (foie, rein, poumon, rétine, cerveau) mais stimulent l'angiogénèse mesurée par le test d'implants cornéens. Ces propriétés en font un marqueur spécifique des cellules endothéliales ayant acquis un phénotype angiogénique.

Le VEGF induit l'angiogénèse et la perméabilité. J induit l'angiogénèse mais pas la perméabilité.

Cela pourrait représenter un avantage majeur en thérapeutique des ischémies. Ainsi, il a été montré chez l'homme que le VEGF induit un effet bénéfique sur l'ischémie par angiogénèse et donc reperfusion des territoires ischémiés (Isner J.M., Pieczek A., Schainfeld R., Blair R., Haley L., Asahara T., Rosenfield K., Razvi S., Walsj K., Symes J.F. Clinical evidence of angiogenesis after arterial gene transfer of VEGF 165 in patients with ischemic limb. Lancet, 1996, 348:370-374), mais il est aussi apparu une complication sous la forme d'un oedème sévère du membre traité (dû à l'action du VEGF sur la perméabilité) qui ne devrait pas se produire lors d'un traitement avec Ig2 J.

### Exemple 2 :

### Tumeur du sein :

Des souris sont ovariectomisées, puis on leur greffe un implant d'oestrogène qui ne sera actif que pendant 3 semaines. Des cellules cancéreuses humaines de cancer de sein (MCF7) déclenchent une tumeur qui s'arrête de croître après 3 semaines quand les oestrogènes ne sont plus actifs. Si l'on injecte des anticorps anti-idiotypes de VEGF (agonistes du récepteur flk-1), les tumeurs continuent de croître, mais les anticorps anti-idiotypes de VEGF stimulent la vascularisation.

### Commentaires :

Utilisation des anti-idiotypes de facteurs de croissance angiogéniques en chimiothérapie :

La chimiothérapie anticancéreuse classique vise à ralentir la prolifération cellulaire, donc à diminuer le nombre relatif des cellules à haut potentiel de multiplication, en particulier les cellules cancéreuses, mais aussi des cellules normales comme celles des parois digestives. Par ailleurs, une tumeur ne peut se développer que si elle est vascularisée. Une alternative thérapeutique consisterait à réduire la vascularisation tumorale par vectorisation de toxines ou d'antimitotiques sur ces néovaisseaux à condition de choisir une cible qui ne soit pas présente sur les vaisseaux normaux, ce qui fait l'objet de l'invention.

Toute tumeur ne peut se développer que si elle synthétise et relargue vers les vaisseaux pré-existants des facteurs de croissance angiogéniques qui induisent la migration, la prolifération et la différenciation des cellules endothéliales en néovaisseaux. Parmi les facteurs angiogéniques, le VEGF (vascular endothelial growth factor, c'est-à-dire facteur de croissance endothéliale vasculaire) est actuellement reconnu comme l'acteur majeur des néovascularisations non contrôlées observées dans la progression tumorale, la rétinopathie diabétique ou la polyarthrite rhumatoïde. Or, un facteur de croissance peut induire de nombreux effets différents selon qu'il se lie à l'un ou à l'autre de ses récepteurs, par exemple la prolifération ou la survie. Donc, l'immunoneutralisation peut avoir des effets bénéfiques sur l'inhibition de la néovascularisation, mais aussi des effets indésirables sur la diminution de la survie de l'endothélium sain. Par ailleurs, la demi-vie du VEGF est de 3 minutes. Il ne circule pas et ne peut donc atteindre sa cible après injection par voie systémique.

La construction d'agonistes de récepteurs du VEGF doit donc répondre à deux impératifs de spécificité et de biodisponibilité. Pour cela, on a mis à profit la réponse idiotypique pour obtenir des images internes des domaines de liaison du VEGF à KDR, qui, du fait de sa structure immunoglobulinique, est circulant.

### Résultats obtenus :

La présente invention propose un protocole de fabrication d'agonistes spécifiques de récepteurs de facteurs de croissance utilisables par voies systémiques. On a ainsi obtenu des agonistes spécifiques du récepteur KDR du VEGF.

L'agoniste du récepteur KDR :
- induit la néovascularisation de tumeurs d'adénocarcinomes de prostate et de sein,
- n'induit pas la prolifération indésirables de l'endothélium sain,
- n'induit pas la perméabilité capillaire à la différence du VEGF,
- n'induit pas d'hypotension à la différence du VEGF.

### REFERENCES

Folkman J:How is blond vessel growth regulated in normal and neoplastic tissue. Cancer Res. 1986;46:467-479.

Plouet J, Schilling J, Gospodarowicz D:Isolation and characterization of a newly identified endothelial cell mitogen produced by AtT20. EMBO J., 1989, 8, 3801-3806.

Ferrara N, Henzel WJ:Pituitary follicular cells secrete a nover heparin binding growth factor specific for vascular endothelial cells. Biochem. Bioph. Res. Commun. 1989;161:851-856.

Conn G, Soderman DD, Schaffer MT, Wile M, Hatcher VB, Thomas KA:Purification of a glycoprotein vascular endothelial cell mitogen from a rat glioma-derived cell line. Proc.Natl.Acad.Sci, 1990;87:1323-1327.

Connolly DT, Olander JV, Heuvelman D, Nelson R, Monsell R, Siegel N, Haymore BL, Leimgruber R, Feder J:Human Vascular Permeability Factor. J. Biol. Chem, 1989;264:20017-20024.

Leung DW, Cachianes G, Kuang WJ, Goeddel DW, Ferrara N: Vascular Endothelial Growth Factor is a secreted angiogenic mitogen. Science 1989;246:1306-1309.

Keck PJ, Hauser SD, Krivi G, Sanzo K, Warren T, Feder J, Connolly DT:Vascular Permeability Factor, an Endothelial Cell Mitogen Related to PDGF. Science 1989;246:1309-1312.

Tischer E, Gospodarowicz D, Mitchel R, Silva M, Schilling J, Lau K, Crisp T, Fiddes JC, Abraham JA:Vascular Endothelial Growth Factor: a new member of the Plateled-Derived Growth Factor Gene Family. Biochem. Bioph. Res Commun. 1989:165:1198-1206.

De Vries C, Escobedo J, Ueno H, Houck K, Ferrara N, Lewis LT:The fms-like tyrosine kinase, a receptor for vascular endothelial growth factor. Science 1992;255:989-991.

Terman BI, Vermazen MD, Carrion ME, Dimitrov D, Armellino DC, Gospodarowicz D, Bohlen P:Identification of the KDR tyrosine kinase as a receptor for vascular endothelial growth factor. Biochem, Biophys. Res, Commun, 1992;34:1578-1586.

Millauer B, wizigmann-Voos S, Schnurch H, Martinez R, Moller NPH, Risau W, Ullrich A High affinity VEGF binding and developmental expression suggest Flk-1 as a major regulator of vasculogenesis and angiogenesis. Cell 1993;72:835-846.

Quinn TP, Peters KG, Devries C, Ferrara N, Williams LT:Fetal liver kinase 1 is a receptor for vascular endothelial growth factor and is selectively expressed in vascular endothelium. Proc. Natl. Acad. Sci. 1993; 90:7533-7537.

Peters KG, DeVries C, Williams LT:Vascular endothelial growth factor receptor expression during embryogenesis and tissue repair suggests a role in endothelial differentiation and blood vessel growth. Proc. Natl. Acad. Sci. 1993; 90:8915-8919.

Kim KJ, Li B, Winer J, Armanini M, Gilett N, Philips HS, Ferrara N:Inhibition of vascular endothelial growth factor-induced angiogenesis suppresses tumor growth in vivo. Nature 1993;362:841-844.

Millauer, B., Shawver, L.K., Plate, K.H., Risau, W., Ullrich, A. 1994. Glioblastoma growth inhibited in vivo by a dominant-negative flk-1 mutant. Nature. 367:576-579.

Folkman, J. 1993. Diagnostic and therapeutic applications of angiogenesis research. C. R. Acad. Sci. Paris. 316:914-916.
Oberg, C., Waltenberger, J., Claesson-Welsh, L., Welsh, M., 1994
Expression of protein tyrosine kinases in islet cells : possible role of the flk-1 receptor for b cell maturation from duct cells.
Growth factors, 10, 115-126,

Yamane A., seetharam, L., Yamaguchi S., Gotoh, N., Takahashi, T., Neufeld, G., Shibuya, M.
A new communication system between hepatocytes and sinusoidal endothelial cells in liver through vascular endothelial growth factor and Flt tyrosine kinase receptor family (Flt-1 and KDR/Flk-1).
Oncogene, 1994, 9, 2683-2690.

Brooks PC, Montgomery AMP, Rosenfeld M, reisfeld RA, Hu T, Klier G, Cheresh DA.
integrin avb3 antagonists promote tumor régression by inducing apoptosis of angiogenic blood vessels.
Cell, 1994, 79, 1157-1164.

## Revendications

1. Utilisation d'anticorps anti-idiotypiques du facteur de croissance endothéliale vasculaire pour la préparation d'un médicament destiné au traitement de pathologies impliquant des cellules endothéliales engagées dans un processus d'angiogénèse, soit pour inhiber l'angiogénèse, soit pour favoriser l'angiogénèse, sans affecter les cellules endothéliales quiescentes.

2. Utilisation d'anticorps anti-idiotypiques du facteur de croissance endothéliale vasculaire pour la préparation d'un médicament destiné au traitement de pathologies impliquant les cellules endothéliales angiogéniques, par stimulation sélective du récepteur KDR.

3. Utilisation d'anticorps anti-idiotypiques du facteur de croissance endothéliale vasculaire selon l'une des revendications 1 ou 2 pour la préparation d'un médicament destiné au traitement de pathologies impliquant des cellules endothéliales engagées dans un processus d'angiogénèse, pour inhiber l'angiogénèse, sans affecter les cellules endothéliales quiescentes, lequel anticorps anti-idiotypique est couplé à une toxine dont la fonction est de bloquer la traduction des protéines ou lequel anticorps anti idiotypique est sous forme de fragment Fab.

4. Utilisation d'anticorps anti-idiotypiques du facteur de croissance endothéliale vasculaire selon l'une des revendications 1 ou 2, pour la préparation d'un médicament destiné au traitement de pathologies impliquant des cellules endothéliales engagées dans un processus d'angiogénèse pour favoriser l'angiogénèse, sans affecter les cellules endothéliales quiescentes.

5. Utilisation d'anticorps anti-idiotypiques du facteur de croissance endothéliale vasculaire selon la revendication 3, dans lequel l'anticorps est couplé à une toxine choisie parmi la saporine, la ricine ou bien un élément radioactif tel que l'iode 125 ou 131.

6. Utilisation d'anticorps anti-idiotypiques du facteur de croissance endothéliale vasculaire pour la préparation d'un médicament destiné à stimuler l'angiogénèse physiologique pour augmenter la vitesse de la formation des vaisseaux sanguins au cours de la cicatrisation, de la maturation du corps jaune de l'ovaire ou stimuler l'angiogénèse au cours de pathologies obstructives des vaisseaux afin de reperfuser des territoires ischémiés lors de thrombose vasculaire.

7. Utilisation d'anticorps anti-idiotypiques du facteur de croissance endothéliale vasculaire associés à une toxine ou de fragment Fab d'anticorps anti-idiotypique pour la préparation d'un médicament destiné au traitement de pathologies nécessitant l'inhibition de l'angiogénèse telles que cancer, rétinopathies diabétiques et le rejet de greffes de cornée.

8. Utilisation d'anticorps anti-idiotypiques du facteur de croissance endothéliale vasculaire selon la revendication 1, pour la préparation d'un produit de diagnostic de pathologies impliquant des cellules endothéliales engagées dans un processus d'angiogénèse.

9. Anticorps anti-idiotypique du facteur de croissance endothéliale vasculaire **caractérisé en ce qu'**il est un ligand du récepteur humain KDR ou du récepteur murin Flk-1 et qu'il n'est pas un ligand de flt.

10. Anticorps anti-idiotypique du facteur de croissance endothéliale vasculaire **caractérisé en ce qu'**il présente les propriétés suivantes :
- il est spécifique vis-à-vis de KDR,
- il est circulant,
- il présente une durée de demi-vie d'environ 23 jours, notamment d'environ 21 jours, et en particulier de 22,5 jours,
- il induit la phosphorylation sur une tyrosine d'une protéine de 200 kDa,
- il induit la prolifération des cellules endothéliales vasculaires,
- il n'induit pas la migration de cellules endothéliales,
- il stimule l'angiogénèse,
- il ne provoque pas d'hypotension artérielle,
- il n'affecte pas la perméabilité des vaisseaux.

11. Fragment Fab de l'anticorps anti-idiotypique selon la revendication 9.

12. Complexe entre un anticorps anti-idiotypique selon la revendication 9 et une toxine, en particulier choisie parmi la saporine, la ricine ou bien entre un anticorps anti-idiotype selon la revendication 9 et un élément radioactif tel que l'iode 125 ou 131.

13. Anticorps anti-idiotypique selon la revendication 9, susceptible d'être obtenu selon le procédé suivant :
- on injecte du VEGF purifié chez un animal, notamment un lapin,
- on prélève le sang pour récupérer les Ig purifiées contenant des IgG anti-VEGF spécifiques, par exemple par chromatographie d'affinité pour la protéine A, puis dans une étape éventuelle, on purifie les IgG anti-VEGF spécifiques à partir des Ig purifiées, par exemple par chromatographie d'affinité pour le VEGF,
- on injecte les susdites Ig purifiées ou les susdites IgG anti-VEGF purifiées chez l'animal de la même espèce que celui utilisé lors de l'injection de VEGF, notamment dans les ganglions poplités de lapin de même origine que celui utilisé lors de l'injection de VEGF,
- on prélève le sang pour récupérer les Ig totales, par exemple par la protéine A, puis pour soumettre les Ig totales à deux immunoadsorbtions :
. une immunoadsorbtion sur une colonne d'affinité préparée avec les Ig préimmunes du lapin qui a servi à faire les IgG anti-VEGF, pour éliminer les anticorps anti-allotypes ou isotypes,
. une immunoadsorbtion sur une colonne d'affinité préparée avec des IgG anti-VEGF, pour purifier les anti-idiotypes.

14. Procédé de préparation d'un anticorps anti-idiotypique selon la revendication 9, **caractérisé en ce que** :
- on injecte du VEGF purifié chez un animal, notamment un lapin,
- on prélève le sang pour récupérer les Ig purifiées contenant des IgG anti-VEGF spécifiques, par exemple par chromatographie d'affinité pour la protéine A, puis dans une étape éventuelle, on purifie les IgG anti-VEGF spécifiques à partir des Ig purifiées, par exemple par chromatographie d'affinité pour le VEGF,
- on injecte les susdites Ig purifiées ou les susdites IgG anti-VEGF purifiées chez l'animal de la même espèce que celui utilisé lors de l'injection de VEGF, notamment dans les ganglions poplités de lapin de même origine que celui utilisé lors de l'injection de VEGF,
- on prélève le sang pour récupérer les Ig totales, par exemple par la protéine A, puis pour soumettre les Ig totales à deux immunoadsorbtions :
. une immunoadsorbtion sur une colonne d'affinité préparée avec les Ig préimmunes du lapin qui a servi à faire les IgG anti-VEGF, pour éliminer les anticorps anti-allotypes ou isotypes,
. une immunoadsorbtion sur une colonne d'affinité préparée avec des IgG anti-VEGF, pour purifier les anti-idiotypes.

15. Compositions pharmaceutiques **caractérisées en ce qu'**elles comprennent à titre de substance active un anti-idiotype selon la revendication 9 ou 10, ou le fragment Fab selon la revendication 11 ou le complexe selon la revendication 12.

## Claims

1. Use of anti-idiotypic vascular endothelial growth factor antibodies for the preparation of a medicament for treatment of pathologies involving endothelial cells involved in an angiogenesis process, either to inhibit the angiogenesis or to promote the angiogenesis, without affecting quiescent endothelial cells.

2. Use of anti-idiotypic vascular endothelial growth factor antibodies for the preparation of a medicament for treatment of pathologies involving angiogenic endothelial cells by selective stimulation of the KDR receptor.

3. Use of anti-idiotypic vascular endothelial growth factor antibodies according to one of claims 1 or 2 for the preparation of a medicament for treatment of pathologies involving endothelial cells involved in an angiogenesis process, to inhibit the angiogenesis, without affecting quiescent endothelial cells, which anti-idiotypic antibody is coupled to a toxin, the function of which is to block translation of proteins, or which anti-idiotypic antibody is in the form of an Fab fragment.

4. Use of anti-idiotypic vascular endothelial growth factor antibodies according to one of claims 1 or 2 for the preparation of a medicament for treatment of pathologies involving endothelial cells involved in an angiogenesis process, to promote the angiogenesis, without affecting quiescent endothelial cells.

5. Use of anti-idiotypic vascular endothelial growth factor antibodies according to claim 3, in which the antibody is coupled to a toxin chosen from saporin and ricin or a radioactive element, such as iodine-125 or -131.

6. Use of anti-idiotypic vascular endothelial growth factor antibodies for the preparation of a medicament for treatment for stimulation of physiological angiogenesis, to increase the speed of formation of blood vessels in the course of cicatrization or maturation of the corpus luteum of the ovary, or for stimulation of angiogenesis in the course of obstructive pathologies of vessels, in order to reperfuse regions rendered ischaemic during vascular thrombosis.

7. Use of anti-idiotypic vascular endothelial growth factor antibodies associated with a toxin or of the Fab fragment of anti-idiotypic antibodies for the preparation of a medicament for treatment of pathologies requiring inhibition of angiogenesis, such as cancer, diabetic retinopathies and rejection of corneal grafts.

8. Use of anti-idiotypic vascular endothelial growth factor antibodies according to claim 1 for the preparation of a product for diagnosis of pathologies involving endothelial cells involved in an angiogenesis process.

9. Anti-idiotypic vascular endothelial growth factor antibody, **characterized in that** it is a ligand of the human KDR receptor or of the murine Flk-1 receptor and **in that** it is not a ligand of flt.

10. Anti-idiotypic vascular endothelial growth factor antibody, **characterized in that** it has the following properties:
- it is specific to KDR,
- it is circulating,
- it has a half-life of about 23 days, especially about 21 days, and in particular 22.5 days,
- it induces phosphorylation on a tyrosine of a protein of 200 kDa,
- it induces proliferation of vascular endothelial cells,
- it does not induce migration of endothelial cells,
- it stimulates angiogenesis,
- it does not cause arterial hypotension,
- it does not affect the permeability of vessels.

11. Fab fragment of the anti-idiotypic antibody according to claim 9.

12. Complex between an anti-idiotypic antibody according to claim 9 and a toxin, in particular chosen from saporin and ricin, or between an anti-idiotypic antibody according to claim 9 and a radioactive element, such as iodine-125 or -131.

13. Anti-idiotypic antibody according to claim 9 which can be obtained by the following process:
- purified VEGF is injected into an animal, in particular a rabbit,
- blood is withdrawn to recover purified Ig containing specific anti-VEGF IgG, for example by affinity chromatography for protein A, and then in a possible stage the specific anti-VEGF IgG are purified from the purified Ig, for example by affinity chromatography for VEGF,
- the abovementioned purified Ig or the abovementioned purified anti-VEGF IgG are injected into an animal of the same species as that used for injection of the VEGF, in particular into the popliteal ganglions of a rabbit of the same origin as that used for injection of the VEGF,
- blood is withdrawn to recover the total Ig, for example by protein A, and then to subject the total Ig to two immunoadsorptions:
. an immunoadsorption on an affinity column prepared with the pre-immune Ig of the rabbit which has been used to produce the anti-VEGF IgG, to eliminate the anti-allotypic or isotypic antibodies,
. an immunoadsorption on an affinity column prepared with the anti-VEGF IgG, to purify the anti-idiotypes.

14. Process for the preparation of an anti-idiotypic antibody according to claim 9, **characterized in that**:
- purified VEGF is injected into an animal, in particular a rabbit,
- blood is withdrawn to recover purified Ig containing specific anti-VEGF IgG, for example by affinity chromatography for protein A, and then in a possible stage the specific anti-VEGF IgG are purified from the purified Ig, for example by affinity chromatography for VEGF,
- the abovementioned purified Ig or the abovementioned purified anti-VEGF IgG are injected into an animal of the same species as that used for injection of the VEGF, in particular into the popliteal ganglions of a rabbit of the same origin as that used for injection of the VEGF,
- blood is withdrawn to recover the total Ig, for example by protein A, and then to subject the total Ig to two immunoadsorptions:
. an immunoadsorption on an affinity column prepared with the pre-immune Ig of the rabbit which has been used to produce the anti-VEGF IgG, to eliminate the anti-allotypic or isotypic antibodies,
. an immunoadsorption on an affinity column prepared with the anti-VEGF IgG, to purify the anti-idiotypes.

15. Pharmaceutical compositions, **characterized in that** they comprise, as active substance, an anti-idiotypic antibody according to claim 9 or 10, or the Fab fragment according to claim 11, or the complex according to claim 12.

## Patentansprüche

1. Verwendung von Anti-Idiotyp-Antikörpern gegen den vaskulären Endothelwachstumsfaktor zur Herstellung eines Medikamentes, das zur Behandlung von Krankheiten bestimmt ist, an denen Endothelzellen beteiligt sind, die in einen Prozess der Angiogenese einbezogen sind, entweder um die Angiogenese zu inhibieren, oder um die Angiogenese zu begünstigen, ohne die ruhenden Endothelzellen zu beeinträchtigen.

2. Verwendung von Anti-Idiotyp-Antikörpern gegen den vaskulären Endothelwachstumsfaktor zur Herstellung eines Medikaments, das zur Behandlung von Krankheiten bestimmt ist, an denen gefäßbildende Endothelzellen beteiligt sind, durch selektive Stimulation des Rezeptors KDR.

3. Verwendung von Anti-Idiotyp-Antikörpern des vaskulären Endothelwachstumsfaktors gemäß einem der Ansprüche 1 oder 2 zur Herstellung eines Medikaments, das zur Behandlung von Krankheiten bestimmt ist, bei denen Endothelzellen beteiligt sind, die in einem Prozess der Angiogenese einbezogen sind, um die Angiogenese zu inhibieren, ohne die ruhenden Endothelzellen zu beeinträchtigen, wobei der Anti-Idiotyp-Antikörper an ein Toxin gekoppelt ist, dessen Funktion es ist, die Proteinsynthese zu blockieren oder wobei der Anti-Idiotyp-Antikörper ein F_{ab}-Fragment ist.

4. Verwendung von Anti-Idiotyp-Antikörpem des vaskulären Endothelwachstumsfaktors gemäß einem der Ansprüche 1 oder 2, zur Herstellung eines Medikaments, das zur Behandlung von Krankheiten bestimmt ist, bei denen Endothelzellen beteiligt sind, die in einem Prozess der Angiogenese einbezogen sind, um die Angiogenese zu begünstigen, ohne die ruhenden Endothelzellen zu beeinträchtigen.

5. Verwendung von Anti-Idiotyp-Antikörpern des vaskulären Endothelwachstumsfaktors gemäß Anspruch 3, in welchem der Antikörper an ein Toxin gekoppelt ist, das aus Saporin, Ricin oder auch einem radioaktiven Element, wie etwa Jod 125 oder 131, ausgewählt ist.

6. Verwendung von Anti-Idiotyp-Antikörpern des vaskulären Endothelwachstumsfaktors zur Herstellung eines Medikaments, das zur Stimulation der physiologischen Angiogenese, zur Erhöhung der Geschwindigkeit der Bildung von Blutgefäßen im Laufe der Vernarbung, der Reifung des Gelbkörpers des Eierstocks oder zur Stimulation der Angiogenese im Laufe von Krankheiten, die die Gefäße zerstören, um die ischemischen Bereiche bei einer vaskulären Thrombose wieder zu durchströmen, bestimmt ist.

7. Verwendung von Anti-Idiotyp-Antikörpern des vaskulären Endothelwachstumsfaktors, die mit einem Toxin assoziiert sind, oder von einem F_{ab}-Fragment von Anti-Idiotyp-Antikörpern zur Herstellung eines Medikaments, das zur Behandlung von Krankheiten bestimmt ist, bei denen es der Inhibierung der Angiogenese bedarf, wie etwas Krebs, diabetische Retinopathien und die Abstoßung von Cornea-Transplantaten.

8. Verwendung von Anti-Idiotyp-Antikörpern des vaskulären Endothelwachstumsfaktors gemäß Anspruch 1, zur Herstellung eines Produkts zur Diagnose von Krankheiten, bei denen Endothelzellen beteiligt sind, die in einem Prozess der Angiogenese engagiert sind.

9. Anti-Idiotyp-Antikörper des vaskulären Endothelwachstumsfaktors, **dadurch gekennzeichnet, dass** dieser ein Ligand des menschlichen Rezeptors KDR oder des Rezeptors Flk-1 von der Maus ist und, dass dieser kein Ligand des flt ist.

10. Anti-Idiotyp-Antikörper des vaskulären Endothelwachstumsfaktors, **dadurch gekennzeichnet, dass** dieser die folgenden Eigenschaften aufweist:
- dieser gegenüber dem KDR spezifisch ist,
- dieser zirkuliert,
- dieser mit einer Halbwertszeit von ungefähr 23 Tagen, vorzugsweise ungefähr 21 Tagen, und insbesondere 22,5 Tage vorhanden ist,
- dieser die Phosphorylierung eines Tyrosins eines Proteins mit 200 kDa induziert,
- dieser die Proliferation von vaskulären Endothelzellen induziert,
- dieser die Migration von Endothelzellen nicht induziert,
- dieser die Angiogenese stimuliert,
- dieser keine arterielle Hypotonie provoziert,
- dieser die Durchlässigkeit der Gefäße nicht beeinträchtigt.

11. Fab-Fragment des Anti-Idiotyp-Antikörpers gemäß Anspruch 9.

12. Komplex zwischen einem Anti-Idiotyp-Antikörper gemäß Anspruch 9 und einem Toxin, insbesondere ausgewählt aus Saporin, Rizin oder zwischen einem Anti-Idiotyp-Antikörper gemäß Anspruch 9 und einem radioaktiven Element, wie etwa dem Jod 125 oder 131.

13. Anti-Idiotyp-Antikörper gemäß Anspruch 9, erhältlich gemäß dem folgenden Verfahren:
- Injektion von gereinigtem VEGF in ein Tier, insbesondere ein Kaninchen,
- Blutabahme, um die gereinigten IgG wiederzugewinnen, die spezifische IgG Anti-VEGF enthalten, zum Beispiel durch Protein A - Affinitätschromatographie, dann in einem optionalen Schritt teilweise Reinigung der spezifischen IgG Anti-VEGF aus den gereinigten Ig, zum Beispiel durch VEGF- Affinitätschromatographie,
- Injektion der gereinigten Ig oder der gereinigten IgG Anti-VEGF in ein Tier der gleichen Art wie dasjenige, das bei der Injektion des VEGF verwendet wurde, insbesondere in die Kniekehlen-Ganglien des Kaninchen gleichen Ursprungs, wie dasjenige, das bei der Injektion des VEGF verwendet wurde,
- Blutabnahme, um die gesamten Ig wiederzugewinnen, zum Beispiel durch das Protein A, dann um die gesamten Ig in zwei Immunadsorptionen zu unterziehen:
. eine Immunadsorption über eine Affinitätssäule, die mit den Präimmun-Ig des Kaninchen hergestellt wurde, der zum Herstellen der IgG Anti-VEGF dient, um die Anti-Allotyp oder Isotyp-Antikörper zu eliminieren,
. eine Immunadsorption über eine Affinitätssäule, die mit den IgG Anti-VEGF hergestellt wurde, um die Anti-Idiotypen zu reinigen.

14. Verfahren zur Herstellung von Anti-Idiotyp-Antikörpern gemäß Anspruch 9, **dadurch gekennzeichnet, dass**:
- Injektion von gereinigten VEGF in ein Tier, insbesondere in ein Kaninchen,
- Blutabnahme, um die gereinigten Ig wiederzugewinnen, die spezifische IgG Anti-VEGF enthalten, zum Beispiel durch Protein A - Affinitätschromatographie, dann in einem optionalen Schritt die teilweise Reinigung der spezifischen IgG Anti-VEGF aus den gereinigten Ig, zum Beispiel durch VEGF-Affinitätschromatographie,
- Injektion der gereinigten Ig oder IgG Anti-VEGF in das Tier der gleichen Art, wie dasjenige, das bei der Injektion des VEGF verwendet wurde, insbesondere in die Kniekehlen-Ganglien des Kaninchen des gleichen Ursprungs, wie dasjenige, das bei der Injektion des VEGF verwendet wurde,
- Blutabnahme, um die gesamten Ig wiederzugewinnen, zum Beispiel durch das Protein A, dann um die gesamten Ig zwei Immunadsorptionen zu unterziehen:
. eine Immunadsorption über eine Affinitätssäule, die mit den Präimmun-IgG des Kaninchen hergestellt wurde, der zum Herstellen der IgG Anti-VEGF dient, um die Anti-Allotyp-oder Anti-Isotyp-Antikörper zu eliminieren,
. eine Immunadsorption über eine mit den IgG Anti-VEGF hergestellte Affinitätskolonne, um die Anti-Idiotypen zu reinigen.

15. Pharmazeutische Zusammensetzung, **dadurch gekennzeichnet, dass** diese als aktive Substanz ein Anti-Idiotyp gemäß Anspruch 9 oder 10, oder F_{ab}-Fragment gemäß Anspruch 11 oder den Komplex gemäß Anspruch 12 umfasst.
